(19) Europäisches Patentamt

European Patent Office

Office européen des brevets

(11) **EP 1 256 347 A1**

(12) **EUROPÄISCHE PATENTANMELDUNG**

(43) Veröffentlichungstag:
**13.11.2002 Patentblatt 2002/46**

(51) Int Cl.[7]: **A61K 31/718**, A61P 1/00

(21) Anmeldenummer: **01810456.2**

(22) Anmeldetag: **10.05.2001**

| | |
|---|---|
| (84) Benannte Vertragsstaaten:<br>**AT BE CH CY DE DK ES FI FR GB GR IE IT LI LU MC NL PT SE TR**<br>Benannte Erstreckungsstaaten:<br>**AL LT LV MK RO SI**<br><br>(71) Anmelder: **Swiss Caps Rechte und Lizenzen AG**<br>**9533 Kirchberg (CH)** | (72) Erfinder: **Kokkinis, Georg**<br>**9306 Freidorf (CH)**<br><br>(74) Vertreter: **Wenger, René et al**<br>**Hepp, Wenger & Ryffel AG**<br>**Friedtalweg 5**<br>**9500 Wil (CH)** |

(54) **Verwendung von Stärke zur Herstellung eines Mittels für eine therapeutische Behandlung**

(57) Es wird die Verwendung von Stärke als ruktations- bzw. flatulenzhemmendes Mittel vorgeschlagen und zwar vorteilhaft für die Herstellung einer einzeldosierten Darreichungsform für Pharmazeutika oder Diätetika. Besonders vorteilhaft eignen sich dabei Weichkapseln aus einer homogenisierten, Stärke enthaltenden Masse.

**Beschreibung**

Die vorliegende Erfindung bezieht sich auf die Verwendung von Stärke zur Herstellung eines Mittels für eine therapeutische Behandlung, nämlich die Herstellung eines ruktationshemmenden bzw. eines flatulenzhemmenden Mittels.

Die Verwendung von Stärke ist in der Pharmazie seit langem bekannt und gebräuchlich. So dienen beispielsweise Stärkepulver, Stärkeschleime oder modifizierte Stärken als Spreng- und Füllmittel für Tabletten, als Gleit- und Füllmittel für Puder und vieles mehr (Römpp Chemielexikon, 9. Auflage, 1993).

Auch als Hüllmaterial für pharmazeutische Kapseln, namentlich für Steckkapseln, ist Stärke seit langem bekannt (z.B. EP-A-118 240). Steckkapseln eignen sich primär für die Verkapselung von Pulvern oder Granulaten, jedoch weniger für Flüssigkeiten. Wirkstoffe in flüssiger oder feste Wirkstoffe in eine pastöse Matrix eingebettet werden mehrheitlich in Weichkapseln abgefüllt. Dazu wird meistens das sogenannte Rotary-Die-Verfahren angewendet, bei dem die Kapsel in einem einzigen Arbeitsgang aus endlosen Materialbändern gebildet und gefüllt wird. Ausschliesslich oder überwiegend aus Stärke bestehende Weichkapseln waren bisher nicht bekannt. Als traditionelles Verkapselungsmaterial wurde hier bis anhin Gelatine eingesetzt.

Ein Teil der in Weichkapseln abgefüllten Stoffe sind schlecht verträglich oder haben einen ausgesprochen schlechten Geschmack. Sie führen ausserdem zur Bildung von Gasen im Magen-Darm-Trakt. Dies äussert sich durch unangenehmes Aufstossen (Ruktation) oder Flatulenz. (Zur Definition siehe Pschyrembel Klinisches Wörterbuch 256. A., Berlin-New York 1990). Durch die EP-A 317 510 ist es zwar bereits bekannt, Kapseln aus einem Bio-Polymer mit einer geruchsbindenden Schicht zu versehen. Damit wird aber lediglich ein Problem gelöst, das sich bei der Lagerung oder unmittelbar bei der Einnahme der Kapseln ergibt. Die unerwünschten Nebeneffekte werden damit im Zusammenhang mit bekannten Kapseln nicht beseitigt. Ausserdem ist nach Zerfall der Hülle und Freigabe des Inhalts die Wirksamkeit nicht mehr oder nur unzureichend gegeben.

Es hat sich nun überraschend gezeigt, dass Stärke oder ein Stärke enthaltendes Gemisch bei ausreichender Dosierung als ruktations- bzw. flatulenzhemmendes Mittel verwendbar ist. Stärke eignet sich daher besonders vorteilhaft zur Herstellung einer einzel dosierten Darreichungsform für beliebige Pharmazeutika oder Diätetika. In bestimmten Fällen wäre aber auch ausschliesslich Stärke oder ein Stärke enthaltendes Gemisch in der Form von Pulver, Tabletten, Pellets, Kapseln oder als Schleim, als ruktationshemmendes bzw. flatulenzhemmendes Mittel denkbar.

Für flüssige oder pastöse Pharmazeutika bzw. Diätetika, ist die Darreichungsform vorteilhaft eine Kapsel, insbesondere eine Weichkapsel, wobei wenigstens die Kapselhülle Stärke enthält bzw. überwiegend aus Stärke besteht. Die eingangs erwähnten störenden Körperfunktionen können nicht nur bei freiverkäuflichen und rezeptpflichtigen Arzneimitteln, sondern auch bei Nahrungsergänzungsmitteln auftreten. Die Kapselhülle aus Stärke wirkt dabei bereits vor der Zersetzung der Kapsel im Magen-Darm-Trakt prophylaktisch. Als besonders vorteilhaft hat sich die Verwendung einer Stärkekapsel im Zusammenhang mit schwerverdaulichen und/oder übelriechenden Stoffen wie z.B. Fischöl, Lebertran, Zwiebel, Bärlauch oder Knoblauch erwiesen.

Bei einer Gruppe von Versuchspersonen wurden besonders hervorragende Ergebnisse erzielt durch die Verwendung einer homogenisierten, Stärke enthaltenden Masse, welche mindestens eine im wesentlichen amorphe Stärke enthält, die vorzugsweise in einem Gewichtsbereich von 45 bis 80 Gewichtsprozent bezogen auf das Gesamtgewicht der Masse vorliegt. Der Amylopektingehalt ist vorzugsweise grösser oder gleich 50 Gewichtsprozent bezogen auf das Gewicht der wasserfreien Stärke. Die Masse enthält weiterhin Wasser, mindestens einen organischen Weichmacher in einem Anteil von mindestens 12 Gewichtsprozent bezogen auf das Gewicht der wasserfreien Stärke, wobei der Staudinger-Index der Stärke in der homogenisierten Masse mindestens 40 ml/g beträgt. Weichkapseln aus einer derartigen Masse werden vorzugsweise im Rotary-Die-Verfahren hergestellt, wobei die entsprechenden Materialbänder durch ein spezielles Extrusionsverfahren erzeugt werden. Einzelheiten zu dieser Masse und zum Herstellungsverfahren sind in der internationalen Patentanmeldung PCT/CH00/00616 beschrieben, die nachstehend auszugsweise wie folgt wiedergegeben und in den Offenbarungsinhalt der vorliegenden Anmeldung eingeschlossen wird:

Verfahren zum Herstellen eines Stärke enthaltenden Formkörpers, insbesondere einer Weichkapsel mit einteiliger Kapselhülle, wobei das Verfahren folgende Schritte umfasst

a) Überführung einer Mischung enthaltend mindestens eine Stärke, Wasser, und mindestens einen organischen Weichmacher, unter Erhitzen und Kneten in eine thermoplastisch verarbeitbare, vorzugsweise homogenisierte, Masse in einer ersten Verarbeitungseinrichtung;

b) gegebenenfalls Herstellen eines lagerfähigen Zwischenproduktes, insbesondere eines Granulates nach Abkühlen der in Schritt a) erhaltenen Masse und nachfolgendes Überführen des Zwischenproduktes in eine thermoplastisch verarbeitbare Masse in einer zweiten Verarbeitungseinrichtung;

c) Herstellen wenigstens eines Materialsstranges, insbesondere eines extrudierten Films, am Ausgang der ersten oder gegebenenfalls zweiten Verarbeitungseinrichtung,

d) Umformen des Materialstranges zu einem Formkörper in einem kontinuierlichen oder intermittierenden Formverfahren;

e) gegebenenfalls Trocknen des Formkörper,

wobei die Schritte a) bis c) derart durchgeführt werden, dass in Schritt d) der Staudinger-Index [η] der Stärke in der den Materialstrang bildenden Masse einen Wert von nicht weniger als 40 ml/g, bevorzugt mindestens 50 ml/g und noch bevorzugter mindestens 80 ml/g aufweist. Noch bessere Eigenschaften werden erhalten, wenn der Staudinger-Index der Stärke einen Wert von grösser oder gleich 100 ml/g hat. Die vorteilhaftesten Eigenschaften werden erhalten mit einem Wert des Staudinger-Index der Stärke von grösser oder gleich 130 ml/g. Der Staudinger-Index darf einen maximalen Wert von 1000 ml/g nicht übersteigen. In einer vorteilhaften Ausführungsform übersteigt der Staudinger-Index 700 ml/g und noch bevorzugter 300 ml/g nicht.

Die in Schritt a) eingesetzte Mischung enthält die Stärke vorzugsweise in einem Gewichtsbereich von 45 bis 80 Gew.% bezogen auf das Gesamtgewicht der Mischung.

Der Begriff "einteilig" soll zur Abgrenzung gegenüber den zweiteiligen Kapseln verstanden werden, welche durch Stecken und/oder Verkleben zweier Kapselteile mit übereinandergelegten Aussenkanten erzeugt werden. Die einteilige Kapselhülle kann gänzlich ohne Nahtstelle oder aber, wenn aus Formteilen gebildet, mit verschweisster Nahtstelle, ausgebildet sein.

Der Begriff "Weichkapsel" ist als Produkt der in der Literatur aufgeführten gängigen kontinuierlichen und halbkontinuierlichen, 1-Schritt-Herstellungsverfahren für einteilige Kapseln zu verstehen. Er dient dabei weniger zur Differenzierung des Weichmachergehaltes, da auch Hartkapseln, als Bezeichnung für zusammengefügte zweiteilige Kapseln, einen Weichmachergehalt von bis zu 12% bezogen auf die Gesamtmasse enthalten können.

Die Begriffe "thermoplastisch verarbeitbar, Schmelze und amorph" werden definiert nach Römpp Chemie Lexikon, Hrsg: J. Falbe, M. Regitz, 9. Auflage, 1992, Georg Thieme Verlag, Stuttgart.
Unter dem Begriff Stärke sollen native Stärken, sowie physikalisch und/oder chemisch modifizierte Stärken verstanden werden. Für die in Schritt a) des erfindungsgemässen Verfahrens eingesetzte Mischung sind alle Stärken, unabhängig von der Pflanze aus der sie gewonnen werden, geeignet. In einer bevorzugten Ausführungsform handelt es sich um Stärke, deren Amylopektingehalt über 50% bezogen auf das Gesamtgewicht der wasserfreien Stärke liegt. Als für das Verfahren bevorzugt haben sich physikalisch und/oder chemisch modifizierte Kartoffelstärken erwiesen.

Für die vorliegende Erfindung sind jedoch im weitesten Sinne alle Polyglucane, d.h. 1.4 und/oder 1.6 Poly-α-D-glucane und/oder Abmischungen zwischen diesen geeignet.

In einer bevorzugten Ausführungsform ist die Stärke eine hydroxypropylierte Stärke. Der Substitutionsgrad (DS = degree of substitution) ist dabei im Bereich von 0.01 bis 0.5, vorzugsweise im Bereich von 0.05 bis 0.25 und noch bevorzugter im Bereich von 0.1 bis 0.15. Insbesondere handelt es sich um hydroxypropylierte Kartoffelstärke.

In einer weiteren bevorzugten Ausführungsform handelt es sich bei der Stärke um vorverkleisterte Stärke. Oberhalb einer für jede Stärkeart typischen Temperatur tritt in wässrigen Stärkesuspensionen nach Erreichen eines Höchstquellungsgrades "Lösung" der Stärkekörner ein, d.h. irreversible Desintegration der Stärkekörner. Dieser Vorgang wird auch als "Verkleisterung" bezeichnet. Die Verkleisterung, d.h. die irreversible Quellung der Stärkekörner bei höherer Temperatur bis zum 40-fachen des ursprünglichen Volumens beruht auf einer allmählichen Wasseraufnahme und Lösung von Wasserstoffbrücken-Bindungen, die eine weitere Hydratation bis zur völligen Desintegration des Stärkekorn-Gefüges ermöglicht.

Die Überführung der Stärke enthaltenden Mischung in den thermoplastischen, vorzugsweise homogenisierten Zustand in Schritt a), ebenso wie die danach folgenden Verarbeitungsschritte b) und c), müssen unter Bedingungen erfolgen, die einen unkontrollierten Abbau der Amylose- und Amylopektinmoleküle zu kurzen Bruchstücken verhindern.

Es muss das Zusammenwirken aller Verarbeitungsparameter wie z.B. Temperatur, Druck, Verweilzeit und Knetleistung, während der Schritte a) bis c) berücksichtigt werden, um einen weitgehenden Abbau der Stärkemoleküle zu verhindern. So kann z.B. auch bei relativ hohen Temperaturen ein weitgehender Abbau der Stärkemoleküle vermieden werden, wenn die Verweilzeiten der Stärke enthaltenden Masse bei diesen Temperaturen klein gehalten wird.

In einer bevorzugten Ausführungsform übersteigt die Temperatur der Masse in der ersten und gegebenenfalls zweiten Verarbeitungseinrichtung, sowie beim Herstellen des Materialstranges 160°C , bevorzugt 140°C, noch bevorzugter 120°C und am vorteilhaftesten 90°C nicht. Bei 160°C sollte insbesondere auch der Aufschlussvorgang in Schritt a) in weniger als 5 Minuten, bevorzugt weniger als 3 Minuten abgeschlossen sein.

In einer weiteren bevorzugten Ausführungsform überschreitet die in die Masse durch das Kneten eingebrachte Energie zur Erzeugung einer thermoplastisch verarbeitbaren homogenisierten Masse in Schritt a) bis c) 0,3 kWh/kg, bevorzugt 0,2 kWh/kg und noch bevorzugter 0,175 kWh/kg nicht.

Die Überführung in den thermoplastisch verarbeitbaren Zustand bewirkt eine irreversible Quellung der Stärkekörner, was eine Vorraussetzung dafür ist, dass die Masse in den homogenen Zustand überführt werden kann bzw. auch nach dem Abkühlen homogenisiert vorliegt. Durch die Schritte a) bis c) wird weiterhin eine Masse erzeugt, in der auch im wesentlichen keine kristallinen Bereiche in der Stärke mehr vorhanden sind. Kristalline Bereiche führen im Materialstrang zu Stippenbildung, d.h. zu Inhomogenitäten, die sich besonders dann nachteilig auswirken, wenn der Materialstrang in Schritt c) ein extrudierter Film ist. Mit "im wesentlichen keine kristallinen Bereiche" soll gemeint sein, dass diese soweit zerstört sind, dass eine Beeinträchtigung der bezüglich der Umformung relevanten physikalischen Parameter des extrudierten Materials nicht auf das Vorhandensein kristalliner Bereiche zurückgeführt werden kann.

Unter dem Begriff "homogene Masse/Material" bzw. "homogenisierte Masse/Material" soll somit ein Material oder eine Masse verstanden werden, die an jeder Stelle im Material die im wesentlichen gleichen physikalische Eigenschaften (Parameter) aufweist. Zu geringfügigen Abweichungen kann es an den jeweiligen Materialoder Formteiloberflächen durch Aufnahme von Luftfeuchtigkeit kommen. Homogen bzw. homogenisiert liegt die Masse dann vor, wenn unter dem Mikroskop die Anzahl der noch sichtbaren Stärkekörner im Schnitt unter einem Prozent liegt. Dazu wird die Masse im thermoplastischen Zustand abgekühlt, in dünne Scheiben geschnitten und unter dem Lichtmikroskop analysiert.

Eine homogenisierte Masse/Material wird erhalten unter Überführen der Mischung in den erweichten oder sogar flüssigen Zustand, der in einen thermoplastisch verarbeitbaren Zustand resultiert. Der Grossteil der die Mischung ausmachenden Komponenten (Stärke, organischer Weichmacher, Gleit- und Formtrennmittel) kann dabei im geschmolzenen Zustand vorliegen und bei genügend langer Stand- und/oder Misch (Knet-)dauer wird die Masse an jeder Stelle der Schmelze die im wesentlichen gleichen Eigenschaften oder chemische Zusammensetzung haben (homogene Masse). Dieser homogene Zustand wird auch bei und nach Abkühlen des thermoplastischen Zustands beibehalten. Es treten keine Entmischungsvorgänge ein. Dies sorgt für gleichmässige mechanische Eigenschaften des Formkörpers bei Raumtemperatur.

Der Staudinger-Index [η] oder auch Grenzviskosität steht innerhalb einer polymerhomologen Reihe mit der Molmasse, dem Gewichtsmittel der Molekulargewichtsverteilung, in folgendem Zusammenhang

$$[\eta] = K \times M^{\alpha}$$

wobei $\alpha$ ein von der Molekül-Gestalt abhängiger Exponent und der K-Wert eine von der gelösten Substanz und vom Lösungsmittel abhängige Konstante ist. Der Staudinger-Index ist innerhalb der polymerhomologen Reihe um so grösser, je grösser das Molekulargewicht des Polymeren bei ansonsten unveränderten Parametern ist. Eine Ermittlung der absoluten Molekulargewichte kann die Messung des Staudinger-Index nicht leisten.

Die Bestimmung von absoluten Molekularmassen bei Stärken ist bekanntermassen überaus schwierig und das Ergebnis ist sehr stark abhängig von der verwendeten Messmethode. Dies gilt um so mehr, je verzweigter die Moleküle sind. Die Ergebnisse der absoluten Molekularmassenbestimmung ist deshalb auch für Amylopektin bzw. amylopektinhaltige Stärke mit einem hohen Unsicherheitsgrad versehen. Da die absolute Molekularmassenbestimmung darüber hinaus sehr teuer ist, liefert die Messung des Staudinger-Index schnellere, verlässlichere und dem Zweck angepasstere Werte.

Ohne eine erschöpfende Erklärung zu liefern, wird vermutet, dass sich in erster Linie der Polymerisationsgrad der Amylopektinemoleküle in der eingesetzten Stärke für die Elastizität und damit für eine möglichst hohe Bruchdehnung des in Schritt d) erzeugten Materialstranges verantwortlich zeigt. Eine hohe Bruchdehnung ist insbesondere für einen bahnförmigen Film, der im Rotary-Die-Verfahren zu einer Weichkapsel geformt werden soll, von grosser Bedeutung.

Es besteht die Vorstellung, dass zusätzlich zur inhärenten Elastizität der Stärkegele, die bei genügendem Polymerisationsgrad der sie konstituierenden Amylopektinmoleküle sowieso gegeben ist, auch eine Art "Stärke-Netzwerk" entstehen kann, das durch Verschlingung und Verhakung der Amylopektinmoleküle aufgebaut wird, und durch Verzweigungen des Moleküls unterstützt wird. Aber auch Amylosemoleküle können bei genügend hohem Polymerisationsgrad, an diesem "Stärke-Netzwerk" partizipieren. Auch die chemische Substitution der Stärkehydroxlygruppen unter Ether-, Ester-, Vinyl- und Acetalbildung können vorteilhaft sein, da sie die Ausbildung von Stärkenetzwerken fördern.

Schritt d) und Schritt e) erfolgt unter Bedingungen, die einen weiteren Abbau der Amylose- und Amylopektinmoleküle vermeiden.

Der in Schritt d) oder e) erhaltene Formkörper weist damit den im wesentlichen gleichen Polymerisationsgrad der Stärke auf, der die Schritte a) bis c) bewirkt haben.

Das Vorhandensein dieser Netzwerke und möglicherweise auch das Vorhandensein von analytisch nicht nachweisbaren und auch in Form von Stippenbildung nicht sichtbaren Nanokristallen (analog Weich-PVC) ist anscheinend für das Auftreten eines Gummiplateaus verantwortlich. Das Young`sche Elastizitätsmodul E amorpher, nicht vernetzter Polymere und insbesondere von linearen Polymeren, fällt normalerweise nach dem Durchlaufen des Bereichs der Glasübergangstemperatur mit steigender Temperatur nahezu linear bis auf $0°C$ ab. Die Polymere verhalten sich bei genügend hoher Temperatur wie eine Flüssigkeit. Das Charakteristikum eines Gummiplateaus ist demgegenüber, dass die mechanischen Eigenschaften wie das Young`sche Elastizitätsmodul E, die Bruchdehnung $\varepsilon_B$, die maximale Festigkeit $\sigma_m$, etc. über eine längere Temperaturspanne annähernd konstant und nahezu unabhängig von der Temperatur bleiben. Ein Gummiplateau ist normalerweise nur bei vernetzten (chemischen Vernetzung) Polymeren zu beobachten (vgl. Intruduction to Polymers, Hrsg. R. J. Young, P. A. Lovell, Chapman und Hall, London, 2. Auflage 1991, Seite 344/345). Unerwarteterweise weisen die Massen der vorliegenden Erfindung trotz Ausbleiben einer dreidimensionalen chemischen Vernetzung ein Gummiplateau auf.

Vor diesem Hintergrund können auch die vorteilhaften Eigenschaften eines 1.4 und 1.6 Polyglukans, das mit kurzen linearen Ketten von 1.4 Polyglukanen kokristallisiert sind, verstanden werden. Durch die Kokristalisierung entstehen zum einen weitere Verzweigungen, die sich auf das Ausbilden eines Netzwerkes positiv auswirken und zum anderen nicht sichtbare Nanokristalline-Bereiche. Bevorzugt werden als 1.4 und 1.6 Polyglukanen Amylopektine eingesetzt.

Die erfindungsgemässen und mit dem erfindungsgemässen Herstellungsverfahren erhaltenen Massen zeigen im Temperaturbereich von etwa 20°C bis ca. 80°C mechanische Eigenschaften, wie z. B. $\varepsilon_B$, $\sigma_m$, E, die im Wesentlichen unabhängig von der Temperatur sind. Das Gummiplateau ist für das Umformen und Füllen der Filme in gefüllte Formkörper von ausschlaggebender Bedeutung. So weist das Young sche Elastizitätsmodul E des erfindungsgemässen Stärke enthaltenden Films im Augenblick des Umformens und Befüllens im Rotary-Die-Prozess maximal 2 MPa, bevorzugt maximal 1 MPa auf. Mit anderen Worten, der Film darf dem Fülldruck des Füllmaterials, der letztendlich die Ausformung der Kapselhülle im Rotary-Die-Prozess bewirkt, bei dem durch die Maschine gegebenen Auflagedruck des Füllkeils nicht einen solchen Widerstand entgegensetzen, dass das Füllmaterial zwischen Film und Füllkeil herausläuft. Es ist eben die Temperaturunabhängigkeit von $\varepsilon_B$ und $\sigma_m$ zwischen 40°C und 90°C , die die Verarbeitbarkeit der aus diesen Massen hergestellten Filme zu Weichkapseln im Rotary-Die-Verfahren ermöglicht.

Der Umformungsvorgang des Materialstranges zu einem Formkörper, insbesondere die Umformung eines extrudierten Films in eine einteilige Weichkapsel mit den in der Technik bekannten Verfahren, erfordert Bruchdehnungen des Materialstranges, insbesondere des Films von mindestens 100% im Bereich von 40°C bis 90°C bevorzugt von 60°C bis 80°C. In einer bevorzugten Ausführungsform ist die Bruchdehnung des Materialstranges, insbesondere Films, mindestens 160% und noch bevorzugter mindestens 240%.

Die Festigkeit $\sigma_m$ des Materialstranges, insbesondere des daraus hergestellten Formkörpers muss bei 25°C und 60% relativer Luftfeuchtigkeit wenigstens 2 MPa betragen. In einer bevorzugten Ausführungsform ist $\sigma_m$ grösser oder gleich 3.5 MPa und noch bevorzugter grösser oder gleich 5 MPa. Dieser Wert gewährleistet bei Raumtemperatur eine genügende Stabilität der Kapselhülle (Verpackung, Lagerung, Transportsicherheit und Gebrauch).

Das Befüllen erfolgt jedoch bei erhöhter Temperaturen des Filmes, die einen Fülldruck von nicht mehr als 2 MPa notwendig macht. Dies ist mit einem Young`schen Elastizitätsmodul E von kleiner oder gleich 2 MPa bei Verkapselungstemperatur (40°C bis 90°C) bei der vorliegenden Masse gegeben. Dies wurde bei den Ausführungen zum Gummiplateau bereits erläutert.

Der Gesamtweichmachergehalt der in Schritt a) eingesetzten Mischung beträgt mindestens 12 Gew. % bezogen auf das Gewicht der wasserfreien Stärke. In einer bevorzugten Ausführungsform ist der Gehalt des Weichmachers in einem Bereich von 30 Gew.% bis 60 Gew.% und noch bevorzugter in einem Bereich von 38 Gew.% bis 55 Gew. %.

Durch die erfindungsgemässe Prozessführung gelingt der weitgehende Ausschluss stark abgebauter Oligomere der Stärke. Dies erlaubt hohe Gesamtmengen an Weichmachern in die Masse einzuarbeiten. Die bei den Homogenisierungsmethoden des bisherigen Standes der Technik entstehenden Oligomere enfalten ebenfalls weichmachende Wirkung und die Einarbeitung grosser Mengen an Weichmacher wäre nicht möglich.

Bevorzugt werden solche Weichmacher eingesetzt, die einen Löslichkeitsparameter von gleich oder > 16,3 $(MPa)^{1/2}$ aufweisen. Die organischen Weichmacher werden ausgewählt aus der Gruppe bestehend aus Polyalkoholen, organischen Säuren, Aminen, Säureamiden und Sulfoxiden. Bevorzugt sind Polyalkohole. Aber auch Wasser wirkt als Weichmacher und bildet damit einen Teil des Gesamtweichmachergehalts. Der Wassergehalt der in Schritt a) eingesetzten Mischung liegt in einem Bereich von 6 bis 30Gew.% bezogen auf die Gesamtmischung.

Der Wasseranteil der in Schritt a) eingesetzten Mischung kann im erfindungsgemässen Verfahren in Schritt b) oder c) gezielt verändert werden. Die physikalischen Parameter, die vom Wassergehalt abhängen, können so Veränderungen unterworfen werden.

Der in Schritt a) eingesetzten Mischung kann je nach erforderlichen Eigenschaften des in d) und e) resultierenden Formkörpers noch mindestens ein Zuschlagstoff in einem Gewichtsbereich von 3,5 Gew.% bis 15 Gew.%, bevorzugt von 5 Gew.% bis 8 Gew.% bezogen auf das Gesamtgewicht der Mischung zugesetzt werden. Die Zuschlagstoffe werden ausgewählt aus der Gruppe bestehend aus Carbonaten und Hydrogencarbonaten der Alkali- und Erdalkaliionen, weitere Zerfallshilfen, Füllstoffe, Farbstoffe, Antioxidantien, physikalisch und/oder chemisch modifizierte Biopolymere insbesondere Polysaccharide und pflanzliche Polypeptide.

Opazität der homogenisierten Masse wird z.B. bevorzugt mit dem Zusatz von Titaniumdioxid als Füllstoff erreicht.

Als Zerfallshilfe, für einen schnellen Zerfall der Kapselhülle werden bevorzugt Calciumcarbonat und Amylasen zugesetzt.

Die Gruppe der physikalisch und/oder chemisch modifizierten Biopolymere umfasst Cellulose, insbesondere teilhydroxypropylierte Cellulose, Alginate, Carrageenan, Galactomannane, Glucomannane, Casein.

In einer bevorzugten Ausführungsform enthält die in Schritt a) eingesetzte Mischung zusätzlich ein internes Gleit- und Formtrennmittel, welches ausgewählt ist aus der Gruppe bestehend aus Lecithinen, Mono-, Di- oder Triglyceriden von Speisefettsäuren, Polyglycerinester der Speisefettsäuren, Polyethylenglycolester der Speisefettsäuren, Zuckerester der Speisefettsäuren und Speisefettsäuren.

Das Gleit- und Formtrennmittel ist in der Mischung bevorzugt in einem Bereich von 0 bis 4 Gew.% bezogen auf das Gesamtgewicht der Mischung enthalten. Vorzugsweise wird es der Mischung in 0,5 bis 2 Gew.% und noch bevorzugter in 0,8 bis 1,5 Gew.% zugesetzt. Vorteilhaft ist das Gleit- und Formtrennmittel ausgewählt aus der Gruppe bestehend aus Glycerinmonostearat und Lecithin.

Unter Speisefettsäuren werden die als Säurekomponenten der Triglyceriden natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine grosse Gruppe der Fettsäuren sind gesättigte Fettsäuren.

Die Stärkemasse kann im thermoplastisch verarbeitbaren Zustand in Schritt c) mittels Breitschlitzdüse zu einem Stärkefilm bzw. Stärkeband extrudiert werden. Die Masse kann jedoch auch aus dem thermoplastisch verarbeitbaren Zustand ungeformt, abgekühlt, getrocknet und zu einem lagerfähigen (unter Feuchtigkeitsabschluss) Granulat verarbeitet werden. Dieses Granulat steht für eine spätere Verarbeitung zur Verfügung. Optional können der zu Granulaten verarbeiteten Masse auch nur ein Anteil der notwendigen Gleit- bzw. Formtrennmittel, Weichmacher und Zuschlagstoffe zugefügt werden. Es kann z.B. auf die Zugabe der tierischen und/oder pflanzlichen Fette zur Vermeidung unerwünschter Farbeffekte in der ersten Verarbeitungseinrichtung verzichtet werden und diese erst beim Wiederaufschmelzen des Granulates in der zweiten Verarbeitungseinrichtung zumischen.

Die extrudierten Bänder werden nun entweder direkt weiterverarbeitet oder gegebenenfalls zur Lagerung unter Verwendung von Kunststofffolien als Zwischenschicht auf Rollen aufgewickelt. Als geeignetstes Folienmaterial hat sich dabei Polyethylen erwiesen.

Der mittels des erfindungsgemässen Verfahrens erhaltene Stärkefilm kann insbesondere für Herstellung von Weichkapseln auf allen in der Technik bekannten Anlagen zur Herstellung einteiliger Kapseln verarbeitet werden. Als besonders geeignet haben sich kontinuierliche Anlagen und insbesondere der Rotary-Die-Prozess erwiesen. Die Kapselwand wird dabei aus zwei vorab aus einem Stärkefilm herausgestanzten Formteilhälften unter Wärmewirkung bevorzugt grösser oder gleich 50°C verschweisst. Zwei "endlose Stärkefilme" werden durch zwei benachbarte, in gegenläufigem Sinn rotierenden Rollen oder Walzen mit Aussparungen geführt. Während der Stärkefilm durch den Fülldruck der Füllmasse in die Aussparung gepresst und somit die Kapselhälften geformt werden, wird die pump- und spritzbare Kapselfüllung mittels eines Ventils exakt dosiert und über einen Füllkeil in den Einzugszwinkel der Formwalzen eingebracht. Die Form und Grösse der Kapsel ist somit abhängig von den geometrischen Abmessungen der Aussparungen in den Walzen und dem eindosierten Füllvolumen.

Konsequenterweise soll unter dem Begriff Kapsel deshalb nicht nur die typischen Kapselformen verstanden werden, sondern auch jede andere mögliche Form von "Hüllen", wie z.B. Kugeln, Kissen und Figuren. Bis heute existieren zahlreiche Weiterentwicklungen und Abweichungen von diesem grundlegenden Prinzip.

Die mittels des erfindungsgemässen Stärkefilms hergestellten einteiligen Kapselhüllen können zusätzlich beschichtet werden, z.B. um die Freisetzung von Wirksubstanzen zu verzögern.

Die Coextrusion, Beschichtung und das Laminieren des erfindungsgemässen Stärkefilms mit Materialien, deren filmbildende Eigenschaft auf synthetischen und/oder natürlichen Polymeren beruht, verschafft zusätzlich Möglichkeiten bestimmte Eigenschaften der Kapselhülle durch eine Mehrschichtfolie zu gestalten.

Insbesondere lässt sich durch den mehrschichtigen Aufbau eine Stärkefolie herstellen, die auf der Innenseite eine gut schweissbare Beschichtung aufweist, während die Aussenseite derart beschichtet wird, dass eine Retardwirkung des Zerfalls der Kapsel eintritt.

Teil der vorliegenden Erfindung ist weiterhin eine homogenisierte, Stärke enthaltende Masse, welche mindestens eine im wesentlichen amorphe Stärke enthält, die vorzugsweise in einem Gewichtsbereich von 45 bis 80 Gew.% bezogen auf das Gesamtgewicht der Masse vorliegt, die Masse enthält weiterhin Wasser, mindestens einen organischen Weichmacher in einem Anteil von mindestens 12 Gew.% bezogen auf das Gewicht der wasserfreien Stärke, wobei der Staudinger-Index der Stärke in der homogenisierten Masse mindestens 40 ml/g beträgt.

Bevorzugt ist ein Staudinger-Index der Stärke von mindestens 50ml/g, noch bevorzugter mindestens 80 ml/g. Besonders bevorzugt ist ein Staudinger-Index der Stärke von grösser oder gleich 100 ml/g. Noch bessere Eigenschaften werden erhalten mit einem Staudinger-Index der Stärke von grösser oder gleich 130 ml/g . Der Staudinger Index der Stärke darf 1000 ml/g, bevorzugt 700 ml/g und noch bevorzugter 300 ml/g nicht überschreiten.

Vorteilhaft wird eine Stärke mit einem Amylopektingehalt von grösser oder gleich 50 Gew.% bezogen auf das Gewicht der wasserfreien Stärke eingesetzt.

Der Gehalt an organischen Weichmachern liegt vorteilhaft im Bereich von 30 Gew.% bis 60 Gew.

%, bevorzugt in einem Bereich von 38 Gew.% bis 55 Gew.% und noch bevorzugter in einem Bereich von 40 bis 50 Gew.% bezogen auf das Gesamtgewicht der Masse.

Bezüglich der Ausführungsformen der Weichmacher, Stärke und Zuschlagsstoffe wird auf die entsprechenden Ausführungen zum Verfahren verwiesen.

In einer besonderen Ausführungsform enthält der Formkörper einen Wassergehalt von maximal 15 Gew.% bezogen auf das Gesamtgewicht der Masse.

Wenn die Masse als Film ausgebildet ist und für die Herstellung von einteiligen Kapselhüllen im Rotary-Die-Prozess herangezogen werden soll, ist eine Bruchdehnung bei Verkapselungstemperatur von 40°C bis 90°C von mindestens 100% erforderlich, bevorzugt liegt die Bruchdehnung jedoch bei mindestens 160% und noch bevorzugter mindestens 240%.

Der Formkörper, insbesondere die aus dem Film gebildete Weichkapselhülle hat bei 25°C und 60% relativer Luftfeuchtigkeit eine Festigkeit von $\sigma_m$, bevorzugt mindestens 3,5MPa und noch bevorzugter mindestens 5 MPa.

Teil der Erfindung sind weiterhin Formkörper, welche aus der erfindungsgemässen Masse hergestellt werden.

Weiterhin Teil der Erfindung ist eine einteilige Kapselhülle, welche Stärke mit einem Staudinger-Index von mindestens 40 ml/g, bevorzugt von mindestens 50 ml/g und noch bevorzugter von mindestens 80ml/g enthält. Besonders vorteilhaft ist eine Kapsel mit einem Staudinger-Index der Stärke von 100 ml/g und noch besser einen Staudinger-Index von 130ml/g.

Die erfindungsgemässen Massen eignen sich gut für die Herstellung von Mehrkammer- bzw. Zweikammerkapseln wie sie z. B. in WO 00/28976 beschrieben sind. Da der Wassergehalt des Filmes bzw. der Filme gering eingestellt werden kann, treten in den fertigen getrockneten Kapseln, insbesondere in den die Kammern bildenden Trennwänden, nahezu keine Spannungen auf, was die Stabilität der Mehrkammerkapsel im Vergleich zu Mehrkammer-Weichgelatinekapseln wesentlich erhöht.

Beispielsweise lassen sich Zweikammerkapseln realisieren, deren eine Kammer mit Pulver oder Granulat gefüllt ist und deren andere Kammer eine Flüssigkeit enthält

Der Formkörper, insbesondere die Kapselhülle hat eine Dicke im Bereich zwischen 0,1 und 2 mm bevorzugt zwischen 0,2 und 0,6 mm.

In einer weiteren bevorzugten Ausführungsform besteht der Formkörper, insbesondere die Weichkapselhülle aus einem mehrlagigen Film. Mindestens zwei der Filme haben eine unterschiedliche chemische Zusammensetzung.

Die Stärke kann grundsätzlich aus verschiedenen pflanzlichen Rohstoffen wie Kartoffel, Mais, Weizen, Reis oder Maniok gewonnen werden. Als besonders vorteilhaft, insbesondere für die Herstellung von Weichkapseln hat sich jedoch Kartoffelstärke erwiesen.

Selbstverständlich kann die Stärke zur Beeinflussung der Materialeigenschaften und/oder zur Erzeugung zusätzlicher Wechselwirkungen mit einem Wirkstoff auch noch andere Bio-Polymere Stoffe wie z.B. Gummi Arabicum, Agar Agar usw. enthalten.

In einem Ausführungsbeispiel wurde einer ersten Versuchungsgruppe aus 4 Personen jeweils am Morgen nüchtern eine mit einem Knoblauchpräparat gefüllte herkömmliche Gelatinekapsel verabreicht. Einer zweiten aus vier Versuchspersonen bestehenden Gruppe wurde eine erfindungsgemässe Stärkekapsel, mit dem gleichen Knoblauchpräparat gefüllt, gegeben. Auch diese Personengruppe war nüchtern. Drei Personen der ersten Versuchsgruppe klagten über übermässiges Aufstossen circa 30 Minuten nach -Einnahme der Kapsel, während bei der zweiten Gruppe keine der Versuchspersonen über ein solches Symptom klagte.

**Patentansprüche**

1. Verwendung von Stärke zur Herstellung eines ruktationshemmenden Mittels.

2. Verwendung von Stärke zur Herstellung eines flatulenzhemmenden Mittels.

3. Verwendung nach Anspruch 1 oder 2 zur Herstellung einer einzeldosierten Darreichungsform für Pharmazeutika oder Diätetika.

4. Verwendung nach einem der Ansprüche 1 bis 3, **dadurch gekennzeichnet, dass** die Darreichungsform eine Kapsel ist und dass wenigstens die Kapselhülle Stärke enthält.

5. Verwendung nach Anspruch 4, **dadurch gekennzeichnet, dass** die Kapsel eine Weichkapsel ist.

6. Verwendung nach Anspruch 4 oder 5, **dadurch gekennzeichnet, dass** die Kapsel einen schwerverdaulichen und/oder übelriechenden Stoff, insbesondere Fischöl, Lebertran, Zwiebel, Bärlauch oder Knoblauch enthält.

7. Verwendung nach einem der Ansprüche 1 bis 6, **gekennzeichnet durch** eine homogenisierte, Stärke enthaltende Masse, enthaltend vorzugsweise mindestens 45 Gewichtsprozent einer amorphen Stärke mit einem Amylopektingehalt von vorzugsweise grösser oder gleich 50 Gewichtsprozent bezogen auf das Gewicht der wasserfreien Stärke, Wasser, mindestens einen organischen Weichmacher in einem Anteil von mindestens 12 Gewichtsprozent bezogen auf das Gewicht der wasserfreien Stärke, wobei der Wert des Staudinger-Index der Stärke in der homogenisierten Masse mindestens 40 ml/g beträgt.

8.  Verwendung nach einem der Ansprüche 1 bis 7, **dadurch gekennzeichnet, dass** die Stärke eine Kartoffelstärke ist.

9.  Stärke oder Stärke enthaltendes Gemisch als ruktationshemmendes Mittel.

10. Stärke oder Stärke enthaltendes Gemisch als flatulenzhemmendes Mittel.

11. Stärke oder Stärke enthaltendes Gemisch nach Anspruch 9 oder 10 in der Form einer Kapselhülle für ein Pharmazeutika oder ein Diätetika.

12. Stärke oder Stärke enthaltendes Gemisch nach einem der Ansprüche 9 bis 11 **gekennzeichnet durch** eine homogenisierte Masse, enthaltend vorzugsweise mindestens 45 Gewichtsprozent einer amorphen Stärke mit einem Amylopektingehalt von vorzugsweise grösser oder gleich 50 Gewichtsprozent bezogen auf das Gewicht der wasserfreien Stärke, Wasser, mindestens einen organischen Weichmacher in einem Anteil von mindestens 12 Gewichtsprozent bezogen auf das Gewicht der wasserfreien Stärke, wobei der Wert des Staudinger-Index der Stärke in der homogenisierten Masse mindestens 40 ml/g beträgt.

**Europäisches Patentamt**

# EUROPÄISCHER RECHERCHENBERICHT

Nummer der Anmeldung

EP 01 81 0456

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DE 31 22 058 A (BRUMMER JOHANN GEORG DIPL BRAU) 23. Dezember 1982 (1982-12-23) * Zusammenfassung; Beispiel 1 * * Seite 3, Absatz 1 - Seite 5, Absatz 1 * | 1-3,8-10 | A61K31/718 A61P1/00 |
| X | DATABASE BIOSIS 'Online! BIOSCIENCES INFORMATION SERVICE, PHILADELPHIA, PA, US; 1977 MICHAELIS O E IV ET AL: "COMPARISON OF FEEDING UNREFINED AND REFINED STARCH DIETS ON INTESTINAL UPTAKE AND HEPATIC LIPOGENIC ENZYMES IN THE RAT" Database accession no. PREV197865066414 XP002185700 * Zusammenfassung * & JOURNAL OF NUTRITION, Bd. 107, Nr. 12, 1977, Seiten 2171-2177, EN ISSN: 0022-3166 | 1-12 | |
| X | DATABASE BIOSIS 'Online! BIOSCIENCES INFORMATION SERVICE, PHILADELPHIA, PA, US; 1997 ZHOU XIAOHAN ET AL: "Soluble amylose cornstarch is more digestible than soluble amylopectin potato starch in rats." Database accession no. PREV199799662471 XP002185701 * Zusammenfassung * & JOURNAL OF NUTRITION, Bd. 127, Nr. 7, 1997, Seiten 1349-1356, ISSN: 0022-3166 -/-- | 1-12 | RECHERCHIERTE SACHGEBIETE (Int.Cl.7) A61K |

Der vorliegende Recherchenbericht wurde für alle Patentansprüche erstellt

| Recherchenort | Abschlußdatum der Recherche | Prüfer |
|---|---|---|
| DEN HAAG | 14. Dezember 2001 | A. Jakobs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C03)

EP 1 256 347 A1

## EUROPÄISCHER RECHERCHENBERICHT

**Europäisches Patentamt**

Nummer der Anmeldung

EP 01 81 0456

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DATABASE BIOSIS 'Online! BIOSCIENCES INFORMATION SERVICE, PHILADELPHIA, PA, US; 1997 PERIAGO M J ET AL: "Non-starch polysaccharides and in vitro starch digestibility of raw and cooked chick peas." Database accession no. PREV199799454548 XP002185702 * Zusammenfassung * & JOURNAL OF FOOD SCIENCE, Bd. 62, Nr. 1, 1997, Seiten 93-96, ISSN: 0022-1147 | 1-12 | |
| A | EP 0 118 240 A (WARNER LAMBERT CO) 12. September 1984 (1984-09-12) * das ganze Dokument * | 1-12 | |
| X | FR 2 173 678 A (PLET DESIRE) 12. Oktober 1973 (1973-10-12) * das ganze Dokument * | 1-3,9,10 | RECHERCHIERTE SACHGEBIETE (Int.Cl.7) |
| A | IT 1 183 298 B (TORRE A FARMACEUTICI) 22. Oktober 1987 (1987-10-22) * das ganze Dokument * | 3-7 | |
| X | PHILLIPS J ET AL: "Effect of resistant starch on fecal bulk and fermentation-dependent events in humans." AMERICAN JOURNAL OF CLINICAL NUTRITION, (1995 JUL) 62 (1) 121-30. , XP001050441 * Zusammenfassung * * Seite 125, Spalte 1, Absatz 1; Tabelle 5 * | 2,3, 10-12 | |

-/--

Der vorliegende Recherchenbericht wurde für alle Patentansprüche erstellt

| Recherchenort | Abschlußdatum der Recherche | Prüfer |
|---|---|---|
| DEN HAAG | 14. Dezember 2001 | A. Jakobs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

10

| | Europäisches Patentamt | EUROPÄISCHER RECHERCHENBERICHT | Nummer der Anmeldung<br>EP 01 81 0456 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | EP 0 425 450 A (VALENTINE ENTERPRISES INC) 2. Mai 1991 (1991-05-02)<br>* Seite 3, Zeile 11,12 * | 6 | |
| X | NNANNA I A ET AL: "PROTEIN AND STARCH DIGESTIBILITY AND FLATULENCE POTENTIAL OF GERMINATED COWPEAS VIGNA-UNGUICULATA"<br>JOURNAL OF FOOD SCIENCE,<br>Bd. 55, Nr. 1, 1990, Seiten 151-153, 183,<br>XP001041759<br>ISSN: 0022-1147<br>* Zusammenfassung *<br>* Seite 151, Spalte 1, Absatz 3 *<br>* Seite 152, Spalte 1, Absatz 4 - Spalte 2, Absatz 3; Tabelle 2 * | 1-3,<br>7-10,12 | |
| X | BOREJSZO Z ET AL: "REDUCTION OF FLATULENCE-CAUSING SUGARS BY HIGH TEMPERATURE EXTRUSION OF PINTO BEAN HIGH STARCH FRACTIONS"<br>JOURNAL OF FOOD SCIENCE,<br>Bd. 57, Nr. 3, 1992, Seiten 771-773, 777,<br>XP001041770<br>ISSN: 0022-1147<br>* Zusammenfassung * | 1-3,7,9,<br>10 | RECHERCHIERTE SACHGEBIETE (Int.Cl.7) |
| X | DATABASE BIOSIS 'Online!<br>BIOSCIENCES INFORMATION SERVICE,<br>PHILADELPHIA, PA, US; 1988<br>JHA K: "FLATULENCE PRODUCING ABILITY OF PEPTOSTREPTOCOCCUS-PRODUCTUS"<br>Database accession no. PREV199089116447<br>XP002185703<br>* Zusammenfassung *<br>& EGYPTIAN JOURNAL OF MICROBIOLOGY,<br>Bd. 23, Nr. 1, 1988, Seiten 193-197,<br>ISSN: 0301-8172 | 1-3,6,7,<br>9,10 | |

-/--

Der vorliegende Recherchenbericht wurde für alle Patentansprüche erstellt

| Recherchenort | Abschlußdatum der Recherche | Prüfer |
|---|---|---|
| DEN HAAG | 14. Dezember 2001 | A. Jakobs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C03)

| | Europäisches Patentamt | EUROPÄISCHER RECHERCHENBERICHT | | Nummer der Anmeldung |
| --- | --- | --- | --- | --- |
| | | | | EP 01 81 0456 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| --- | --- | --- | --- |
| X | DATABASE BIOSIS 'Online! BIOSCIENCES INFORMATION SERVICE, PHILADELPHIA, PA, US; Dezember 1997 (1997-12) LIU JENN-HUA ET AL: "Enteric-coated peppermint-oil capsules in the treatment of irritable bowel syndrome: A prospective, randomized trial." Database accession no. PREV199800030435 XP002185704 * Zusammenfassung * & JOURNAL OF GASTROENTEROLOGY, Bd. 32, Nr. 6, Dezember 1997 (1997-12), Seiten 765-768, ISSN: 0944-1174 | 1-3,6 | |
| X | PHILLIPS R D ET AL: "COMPOSITION AND FLATULENCE-PRODUCING POTENTIAL OF COMMONLY EATEN NIGERIAN AND AMERICAN LEGUMES" FOOD CHEMISTRY, Bd. 33, Nr. 4, 1989, Seiten 271-280, XP001050443 ISSN: 0308-8146 * das ganze Dokument * | 1-3,7,9, 10 | |
| | | | RECHERCHIERTE SACHGEBIETE (Int.Cl.7) |
| X | FLOURIE B ET AL: "COLONIC BREAKDOWN OF 50 GRAM WHEAT STARCH IN HEALTHY MAN EFFECTS ON SYMPTOMS AND FECAL OUTPUTS" GASTROENTEROLOGY, Bd. 86, Nr. 5 PART 2, 1984, Seite 1078 XP001050563 ISSN: 0016-5085 * Zusammenfassung * | 1-3,7,9, 10,12 | |

-/--

Der vorliegende Recherchenbericht wurde für alle Patentansprüche erstellt

| Recherchenort | Abschlußdatum der Recherche | Prüfer |
| --- | --- | --- |
| DEN HAAG | 14. Dezember 2001 | A. Jakobs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**

## EUROPÄISCHER RECHERCHENBERICHT

Nummer der Anmeldung

EP 01 81 0456

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | TOMLIN J ET AL: "The effect of resistant starch on colon function in humans." BRITISH JOURNAL OF NUTRITION, (1990 SEP) 64 (2) 589-95. , XP001050479 * Zusammenfassung * | 1-3,7,9, 10,12 | |
| X | OLSON A C ET AL: "NUTRIENT COMPOSITION OF AND DIGESTIVE RESPONSE TO WHOLE AND EXTRACTED DRY BEANS" JOURNAL OF AGRICULTURAL AND FOOD CHEMISTRY, Bd. 30, Nr. 1, 1982, Seiten 26-32, XP001041769 ISSN: 0021-8561 * Zusammenfassung; Tabelle 7 * | 1-3,7,9, 10,12 | |

| | | RECHERCHIERTE SACHGEBIETE (Int.Cl.7) |
|---|---|---|

Der vorliegende Recherchenbericht wurde für alle Patentansprüche erstellt

| Recherchenort | Abschlußdatum der Recherche | Prüfer |
|---|---|---|
| DEN HAAG | 14. Dezember 2001 | A. Jakobs |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03 82 (P04C03)

**ANHANG ZUM EUROPÄISCHEN RECHERCHENBERICHT**
**ÜBER DIE EUROPÄISCHE PATENTANMELDUNG NR.**    EP 01 81 0456

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-12-2001

| Im Recherchenbericht angeführtes Patentdokument | | Datum der Veröffentlichung | Mitglied(er) der Patentfamilie | | Datum der Veröffentlichung |
|---|---|---|---|---|---|
| DE 3122058 | A | 23-12-1982 | DE | 3043083 A1 | 01-07-1982 |
| | | | DE | 3122058 A1 | 23-12-1982 |
| EP 0118240 | A | 12-09-1984 | AT | 44975 T | 15-08-1989 |
| | | | AU | 572119 B2 | 05-05-1988 |
| | | | AU | 2465684 A | 23-08-1984 |
| | | | BG | 46154 A3 | 16-10-1989 |
| | | | BR | 8400734 A | 02-10-1984 |
| | | | CA | 1238738 A1 | 28-06-1988 |
| | | | CS | 8401078 A2 | 12-02-1987 |
| | | | CS | 8505547 A2 | 12-02-1987 |
| | | | DD | 218113 A5 | 30-01-1985 |
| | | | DE | 3479129 D1 | 31-08-1989 |
| | | | DK | 76584 A | 19-08-1984 |
| | | | EG | 17995 A | 30-06-1992 |
| | | | EP | 0118240 A2 | 12-09-1984 |
| | | | ES | 529815 D0 | 16-05-1985 |
| | | | ES | 8505385 A1 | 01-09-1985 |
| | | | FI | 840614 A ,B, | 19-08-1984 |
| | | | GR | 82253 A1 | 13-12-1984 |
| | | | HU | 36487 A2 | 30-09-1985 |
| | | | IE | 56888 B1 | 15-01-1992 |
| | | | IL | 70980 A | 31-03-1987 |
| | | | IN | 160476 A1 | 11-07-1987 |
| | | | IN | 165520 A1 | 04-11-1989 |
| | | | JP | 1838984 C | 25-04-1994 |
| | | | JP | 59196335 A | 07-11-1984 |
| | | | KR | 9304937 B1 | 10-06-1993 |
| | | | MX | 170518 B | 27-08-1993 |
| | | | NO | 840598 A ,B, | 20-08-1984 |
| | | | NZ | 207188 A | 10-09-1986 |
| | | | PH | 20759 A | 10-04-1987 |
| | | | PL | 246259 A1 | 13-02-1985 |
| | | | PT | 78123 A ,B | 01-03-1984 |
| | | | RO | 88123 A1 | 30-11-1985 |
| | | | RU | 2042423 C1 | 27-08-1995 |
| | | | TR | 23047 A | 14-02-1989 |
| | | | YU | 28684 A1 | 31-12-1986 |
| | | | US | 4673438 A | 16-06-1987 |
| | | | US | 4738724 A | 19-04-1988 |
| FR 2173678 | A | 12-10-1973 | FR | 2173678 A1 | 12-10-1973 |
| | | | DE | 2304305 A1 | 23-08-1973 |
| | | | NL | 7301740 A | 20-08-1973 |
| IT 1183298 | B | 22-10-1987 | KEINE | | |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 01 81 0456

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-12-2001

| Im Recherchenbericht angeführtes Patentdokument | | Datum der Veröffentlichung | Mitglied(er) der Patentfamilie | | Datum der Veröffentlichung |
|---|---|---|---|---|---|
| EP 0425450 | A | 02-05-1991 | US | 5073384 A | 17-12-1991 |
| | | | AT | 125170 T | 15-08-1995 |
| | | | CA | 2026030 A1 | 20-04-1991 |
| | | | DE | 69021007 D1 | 24-08-1995 |
| | | | DE | 69021007 T2 | 21-03-1996 |
| | | | EP | 0425450 A2 | 02-05-1991 |
| | | | JP | 2104022 C | 06-11-1996 |
| | | | JP | 3151005 A | 27-06-1991 |
| | | | JP | 8015521 B | 21-02-1996 |
| | | | US | RE35893 E | 08-09-1998 |
| | | | US | 5275822 A | 04-01-1994 |

EPO FORM P0461

Für nähere Einzelheiten zu diesem Anhang : siehe Amtsblatt des Europäischen Patentamts, Nr.12/82